# EUROPEAN PATENT APPLICATION

(11) **EP 3 479 808 A1**
(43) Date of publication of application: **08.05.2019**
(21) Application number: 18200660.1
(22) Date of filing: 16.10.2018
(51) Int. Cl.: A61G 7/057, A61G 13/10, A61G 13/12, A61F 7/00, A61G 13/00

(54) **UNDERBODY WARMING SYSTEM WITH FOCAL COOLING**

(30) Priority: 26.10.2017 US 201762577385 P
(71) Applicant: Hill-Rom Services, Inc., Batesville, IN 47006-9167 (US)
(72) Inventor: HOOD, Michael Scott, Batesville, IN 47006-9167 (US); DENHAM, Lansdell, Batesville, IN 47006-9167 (US); LAWRENCE, Robert J, Batesville, IN 47006-9167 (US); LACHENBRUCH, Charles A, Batesville, IN 47006-9167 (US)
(74) Representative: Findlay, Alice Rosemary

(57) **Abstract**

An underbody warming system may include a skin contacting surface configured to be positioned on the skin of a user. A plurality of thermoelectric devices may be provided. A first thermoelectric device of the plurality of thermoelectric devices may have a first temperature to create a first temperature gradient between the skin and the first thermoelectric device. A second thermoelectric device of the plurality of thermoelectric devices may have a second temperature different from the first temperature to create a second temperature gradient between the skin and the thermoelectric device. A first flow path may be configured to allow heat to flow between the skin and the first thermoelectric device. A second flow path may be configured to allow heat to flow between the skin and the second thermoelectric device.

## Description

The subject matter disclosed herein relates generally to an underbody patient warming device. More specifically, the present disclosure is related to a patient warming device having an array of thermoelectric devices to prevent ischemic pressure injuries.

Pressure injuries and deep tissue injury may be caused by excessive pressure, surface shear, moisture, and temperature on a patient's skin over time, for example when a patient uses a hospital bed or other patient support apparatus for an extended period. Protective barrier dressings (e.g., bandages) or surfaces (e.g., mattresses) may be used to prevent pressure injuries by reducing friction, surface shear, and/or moisture on the patient's skin. Cooling of the patient's skin may have a significant impact on prevention and treatment of pressure injuries and tissue damage. Pressure injuries are caused primarily by ischemia which is a combination of tissue deformation (pressure) causing occlusion and reduced supply versus temperature (increased demand). Reduced temperature may reduce moisture accumulation on the skin. Also, reduced temperature reduces tissue metabolic rate, which also reduces the severity of tissue damage. As a result, cooling the patient's skin may allow the skin to tolerate a given pressure for a longer time period. Researchers have hypothesized that if tissue temperature could be reduced to approximately 60° F, the tissue could survive occlusion-level pressures near indefinitely. Existing underbody warming systems heat the bony prominences in contact with the warming surface and thus increase the risk of pressure injury development. Micro-climate management (MCM) layers capable of reducing the temperature of patient support surfaces (e.g. mattresses) are known, but may be incompatible with some underbody warming systems. Underbody warming reduces interference with procedures and access to the body. Unfortunately, existing underbody warming solutions have been shown to increase pressure injuries at the bony prominences.

The present disclosure includes one or more of the following features alone or in any combination.

According to one aspect, an underbody warming system may include a skin contacting surface configured to be positioned on the skin of a user. A plurality of thermoelectric devices may be provided. A first thermoelectric device of the plurality of thermoelectric devices may be positioned adjacent a first section of the skin contacting surface and may have a first temperature different from the temperature of the skin being contacted by the first section of the skin contacting surface to create a first temperature gradient between the skin and the first thermoelectric device. A second thermoelectric device of the plurality of thermoelectric devices may be positioned adjacent a second section of the skin contacting surface and may have a second temperature different from the first temperature and the temperature of the skin being contacted by the second section of the skin contacting surface to create a second temperature gradient between the skin and the thermoelectric device. An insulator may be positioned between the first thermoelectric device and the second thermoelectric device. A first flow path may be configured to allow heat to flow between the skin and the first thermoelectric device. A second flow path may be configured to allow heat to flow between the skin and the second thermoelectric device. In some embodiments, a cushion layer that may include a thermally conductive material may be positioned between the skin contacting surface and the plurality of thermoelectric devices.

In some embodiments, at least one pressure sensor may detect a bony prominence of the user. The second thermoelectric device may be positioned at the bony prominence of the user. Acontroller may selectively operate the second thermoelectric device based on data from the pressure sensor.

Optionally, the first temperature may be greater than the second temperature. The first temperature gradient may provide heat to the skin being contacted by the first section of the skin contacting surface. The second temperature gradient may remove heat from the skin being contacted by the second section of the skin contacting surface. The second section of the skin contacting surface may be configured to be positioned at a bony prominence of the user.

Alternatively or in addition to, at least one of the plurality of thermoelectric devices may include a thermometer to monitor a temperature of at least one of the thermoelectric device and a temperature of the skin. A controller may alter a current to the thermoelectric device in response to the temperature monitored by the thermometer.

It may be desired that the thermoelectric device includes a channel to circulate fluids through a cold sink of the thermoelectric device. A thermal exchanger may circulate fluids to and from the thermoelectric device. The skin contacting surface may be disposed within a surgical platform. A power supply may be positioned on the surgical platform.

According to another aspect, an underbody warming system may include a skin contacting surface configured to be positioned on the skin of a user. A plurality of thermoelectric devices may be provided. A first thermoelectric device of the plurality of thermoelectric devices may be positioned adjacent a first section of the skin contacting surface and may have a first temperature different from the temperature of the skin being contacted by the first section of the skin contacting surface to create a first temperature gradient between the skin and the first thermoelectric device. The first temperature gradient may provide heat to the skin being contacted by the first section of the skin contacting surface. A second thermoelectric device of the plurality of thermoelectric devices may be positioned adjacent a second section of the skin contacting surface and may have a second temperature different from the first temperature and the temperature of the skin being contacted by the second section of the skin contacting surface to create a second temperature gradient between the skin and the thermoelectric device. The second temperature gradient may remove heat from the skin being contacted by the second section of the skin contacting surface. A first flow path may be configured to allow heat to flow between the skin and the first thermoelectric device. A second flow path may be configured to allow heat to flow between the skin and the second thermoelectric device. A cushion layer may be positioned between the skin contacting surface and the plurality of thermoelectric devices. In some embodiments, the second section of the skin contacting surface may be configured to be positioned at a bony prominence of the user.

Optionally, at least one of the plurality of thermoelectric devices may include a thermometer to monitor a temperature of at least one of the flexible substrate and the skin. A controller may alter a current to the thermoelectric device based on the temperature monitored.

It may be desired that the thermoelectric device includes a channel to circulate fluids through a cold sink of the thermoelectric device. A thermal exchanger may circulate fluids to and from the thermoelectric device. The skin contacting surface may be disposed within a surgical platform. A power supply may be positioned on the surgical platform.

A method of providing localized cooling to the skin of an underbody warming system user is disclosed which includes positioning a skin contacting surface on the skin of the user. The method may also include positioning a first thermoelectric device adjacent a first section of the skin contacting surface. The method may also include altering a temperature of the first thermoelectric device to create a first temperature gradient between the skin and the first thermoelectric device. The method may also include positioning a second thermoelectric device adjacent a second section of the skin contacting surface. The method may also include altering a temperature of the second thermoelectric device to create a second temperature gradient between the skin and the second thermoelectric device. The method may also include providing a first flow path through the first thermoelectric device to allow heat to flow between the skin and the first thermoelectric device. The method may also include providing a second flow path through the second thermoelectric device to allow heat to flow between the skin and the second thermoelectric device.

In some embodiments, the method may include positioning a cushion layer between the skin contacting surface and the first thermoelectric device. The method may also include positioning the cushion layer between the skin contacting surface and the second thermoelectric device.

Optionally, the method may include altering the temperature of the first thermoelectric device to a temperature that is greater than the temperature of the second thermoelectric device. The method may also include providing heat to the skin being contacted by the first section of the skin contacting surface through the first temperature gradient. The method may also include removing heat from the skin being contacted by the second section of the skin contacting surface through the second temperature gradient. The method may also include positioning the second section of the skin contacting surface at a bony prominence of the user.

It may also be desired that the method include monitoring a temperature of the skin. The method may also include altering a current to the thermoelectric device in response to the temperature monitored by the thermometer. The method may also include circulating fluids from a thermal exchanger through a cold sink of the first thermoelectric device. The method may also include circulating fluids from a thermal exchanger through a cold sink of the second thermoelectric device.

Alternatively, or in addition to, the method may include disposing the skin contacting surface within a surgical platform. The method may also include disposing a power supply on the surgical platform.

The invention will now be further described by way of example with reference to the accompanying drawings, in which:
Fig. 1 is a perspective view of an underbody warming system having a heat exchanger module and integrated into a surgical platform;
Fig. 2 is a cross-sectional view of a rail of the surgical platform shown in Fig. 1 and having an electronic bus to power the underbody warming system;
Fig. 3 is a perspective view of an underbody warming system having a heat exchanger module and integrated into another surgical platform;
Fig. 4 is a schematic diagram of a heat exchanger module in accordance with an embodiment;
Fig. 5 is a top view of a thermoelectric cooler in accordance with an embodiment;
Fig. 6 is a front view of the thermoelectric cooler shown in Fig. 5;
Fig. 7 is a model of a temperature gradient through a heat exchanger module;
Fig. 8 is a graph illustrating temperatures through various thickness of tissue, i.e. skin, fat, muscle, and bone;
Fig. 9 is a perspective view of another embodiment of a surgical platform.

The embodiments described herein relate to devices, systems and methods to warm a patient, while reducing the risk of pressure injuries by cooling the tissue over the bony prominences. The device also allows unrestricted access to the surgical site which is usually facing upward toward the surgeon's field of view. Generally, underbody warming systems heat the bony prominences in contact with the warming surface, thereby increasing the risk of pressure injury development. The devices, systems and methods described herein use focal cooling of the bony prominences to reduce pressure injuries originating in the operating room, which are estimated to account for 40% of hospital-acquired pressure injuries. In other embodiments, the devices, systems and methods described herein may be used outside of the operating, e.g. in a patient room, an ambulance, an emergency transport, or a homecare setting to name a few non-limiting examples.

The described devices, systems and methods are not limited to the specific embodiments described herein. In addition, components of each device, system and/or steps of each method may be practiced independent and separate from other components and method steps, respectively, described herein. Each component and method also can be used in combination with other systems and methods.

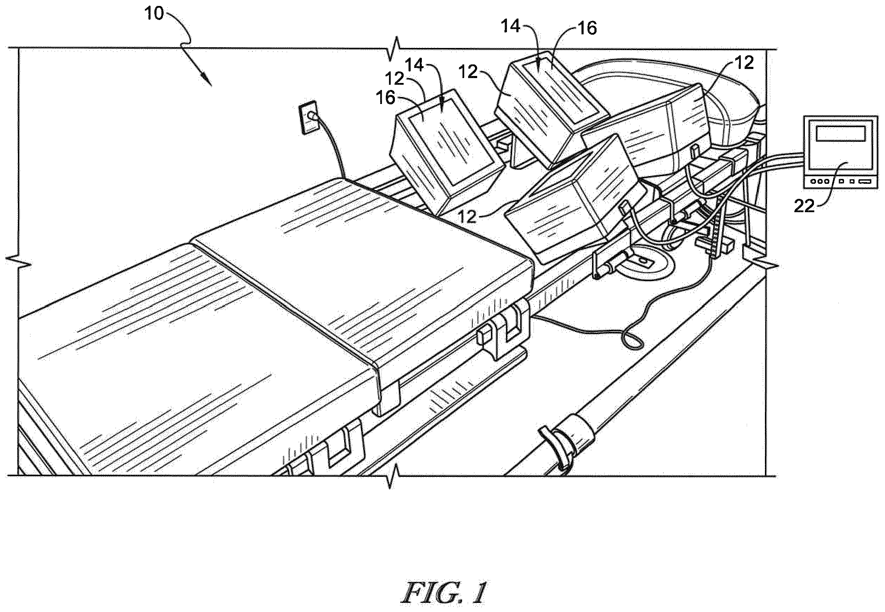

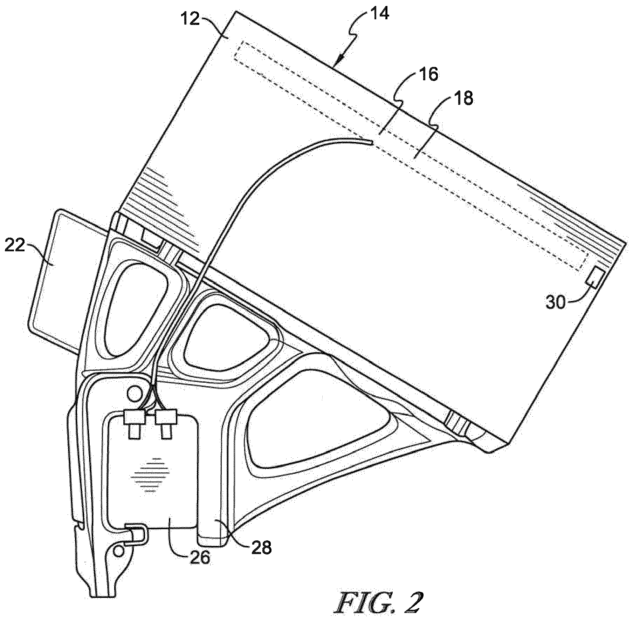

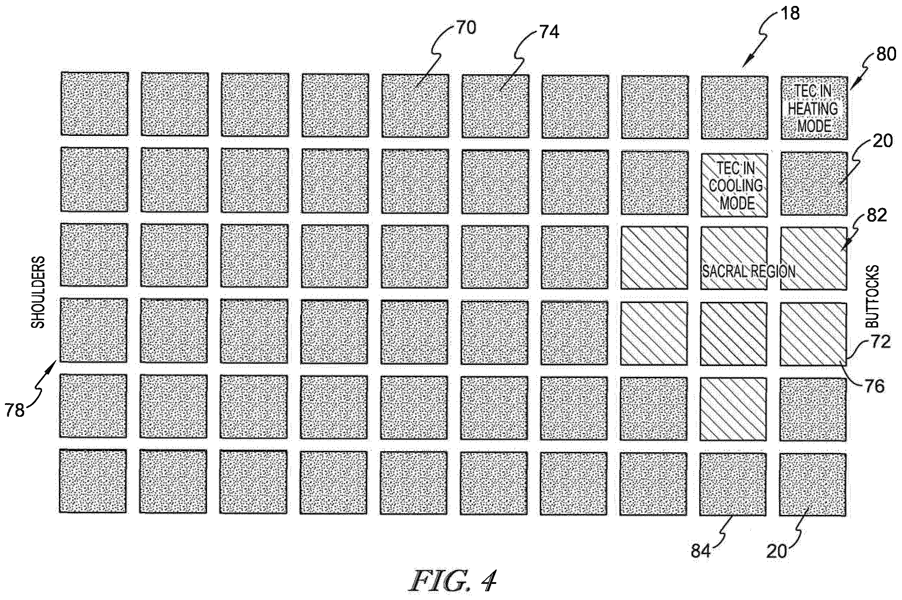

Referring to Figs. 1-2, a surgical platform 10 includes a plurality of patient support cushions 12, which are the areas that have the highest interface pressure with a patient. The cushions 12 include a skin contacting surface 14 that contacts the skin of the patient. The cushion 12 may include a thermally conductive material. An underbody warming device 16 is positioned within at least one of the cushions 12. It should be noted that the underbody warming device 16 may be positioned on any area of the surgical platform 10 that requires warming for a patient, e.g. the upper back, the buttocks, the legs, and/or the arms. A heat exchanger module 18 is positioned within the underbody warming device 16 and includes a plurality of thermoelectric coolers 20 (described in detail with respect to Figs. 4-6). In Fig. 2, the heat exchanger module 18 is illustrated as being positioning within the cushion 12. Optionally, the heat exchanger module 18 may be positioned on top of the cushion 12 and retained on the cushion 12 with straps. In some embodiments, the heat exchanger module 18 may be strapped to the patient. the As described in more detail below, the thermoelectric coolers 20 may be heated to provide heat to a user and/or the thermoelectric coolers 20 may be cooled to provide selective cooling to the user to prevent pressure injuries. The thermoelectric coolers 20 are be powered through removable cords that plug into a thermal controller 22 on a support column 24 of the surgical platform 10. Alternatively, power busses 26 may be integrated into table rails 28 of the surgical platform 10 to eliminate the need for cords, as illustrated in Fig. 2. The surgical platform 10 may include a plurality of power buses 26 positioned adjacent the heat exchanger modules 18 within the surgical platform 10. Alternatively, the power buses 26 may extend a length of the rail 28 and include a plurality of ports to receive cables from the individual heat exchanger modules 18. The warming thermoelectric coolers 20 may be attached to the unsupported areas of the patient with elastic straps. The heating thermoelectric coolers 20 should cover the width of the surgical platform 10. The length should reach from just above the shoulders to the foot section. The cooling thermoelectric coolers 20 are positioned within the same thermoelectric cooler array, e.g. in the pelvic region, occiput region or heel region.

The controller 22 powers the heat exchanger module 18 to heat and cool the thermoelectric coolers 20. The controller 22 may be a proportional integral derivative controller that provides a feedback loop between the heat exchanger module 18 and the controller 22. For example, temperature sensors 30 may monitor a temperature of the patient's skin adjacent each thermoelectric cooler 20. Optionally, the temperature sensors 30 may monitor a temperature of the thermoelectric cooler 20. A signal indicative of the temperature of each thermoelectric cooler 20 is delivered to the controller 22. The controller 22 may then alter a current to any number of thermoelectric cooler 20 to alter the temperature of the thermoelectric coolers 20. The controller 22 is illustrated as being physically integrated into a patient support apparatus 10; however, the controller 22 may be a standalone unit that is electrically coupled to the heat exchanger module 18.

In some embodiments the underbody warming device 16 may be used with a stretcher that is used to transport patients to the operating room and/or post-anesthesia care unit. In some embodiments the underbody warming device 16 may be used in the emergency room on stretchers and procedural chairs for hypothermic patients, in emergency transports, e.g. ambulances, planes, helicopters, or for use with Xport boards and stretchers. The underbody warming device 16 may also be used on the patient floors to optimize patient comfort. The underbody warming device 16 may also be utilized with wheelchairs, chairs, and vehicle seats.

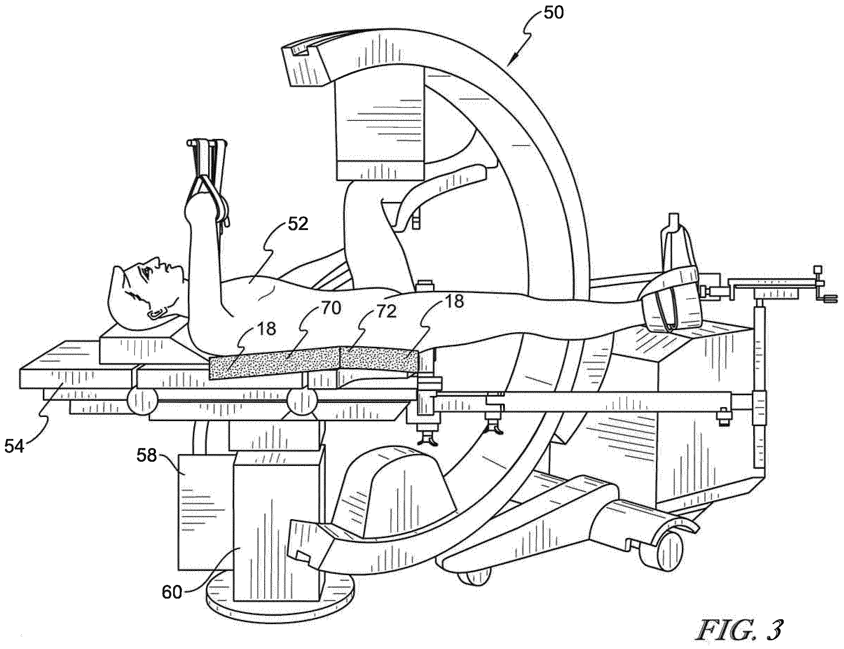

Referring now to Fig. 3, a surgical platform 50 is illustrated with a patient 52 positioned on the cushions 54 of the surgical platform. A heat exchanger module 18 is positioned within the cushions 54 below the patient's back and buttocks. A thermal exchanger 58 for circulating liquids to and from the heat exchanger module 18 is coupled to a stand 60 of the surgical platform 50. Notably, the thermal exchanger 58 may be positioned at any location within the surgical room and fluidly coupled to the heat exchanger module 18. The heat exchanger module 18 includes a heating zone 70 and a cooling zone 72. The heating zone 70 is positioned adjacent the patient's back and the cooling 72 is positioned adjacent the patient's buttocks. The heating zone 70 includes a plurality of thermoelectric coolers 20 that are configured to provide heat to the patient's skin, specifically, the section of the patient's skin positioned on the heating zone 70. The cooling zone 72 includes a plurality of thermoelectric coolers 20 that are configured to removes heat from the patient's skin, specifically, the section of the patient's skin positioned on the cooling zone 72. For example, in the illustrative embodiment, the heating zone 70 provides heat to the patient's back and the cooling zone 72 removes heat from the patient's buttocks.

Generally, the cooling thermoelectric coolers 20 are positioned under the bony prominences, e.g. those around the pelvis including the sacrum, trochanters, ischial tuberosity, and iliac crest, as this area accounts for approximately 55% of pressure injuries originating in the operating room. Other bony prominences may include ankles, greater trochanters, iliac crest, elbows, knees, occiput, nose, forehead and other facial bony prominence, heels, and scapulae, clavicle. In one embodiment, pressure mapping is utilized to detect bony prominences and other areas with a high risk of developing injuries where cooling would be focused. Pressure sensors within the heat exchanger module 18 monitor a degree of pressure being applied to each thermoelectric cooler 20. The controller 22 detects areas of high pressure. High pressure may be determined as a function of the patient's weight, the patient's size, and/or the patient's position. By monitoring areas of high pressure, the controller 22 determines where the patient's bony prominences and other areas of high risk are located. The controller 22 utilizes this information to selectively operate certain thermoelectric coolers 20 at the bony prominence as cooling thermoelectric coolers 20. In some embodiments, if the patient is moved during the operation, the controller 22 may monitor changes in pressure to reconfigure the cooling thermoelectric coolers 20.

Referring to Fig. 4, the heating zone 70 may partially surround the cooling zone 72. Fig. 4 illustrates the heating zone 70 having a first plurality 74 of thermoelectric coolers 20 configured to provide heat. The cooling zone 72 includes a second plurality 76 of thermoelectric coolers 20 configured to remove heat. The first plurality 74 of thermoelectric coolers 20 extend from a shoulder position 78 of the surgical platform to a buttocks position 80 of the surgical platform. The second plurality 76 of the thermoelectric coolers 20 are positioned adjacent the buttocks position 80 and isolated around a sacral position 82 of the surgical table where the patient's sacrum is positioned during surgery. Each of the thermoelectric coolers 20 is surrounded by an insulating material 84, e.g. an insulating metal or polymer, and insulating gel, and/or an insulating fluid. The insulating material 84 facilitates limiting heat loss from the thermoelectric cooler 20. In some embodiments, the first plurality 74 of thermoelectric coolers 20 may be collectively surrounded by an insulating material 84. Likewise, the second plurality 76 of thermoelectric coolers 20 may be collectively surrounded by an insulating material 84.

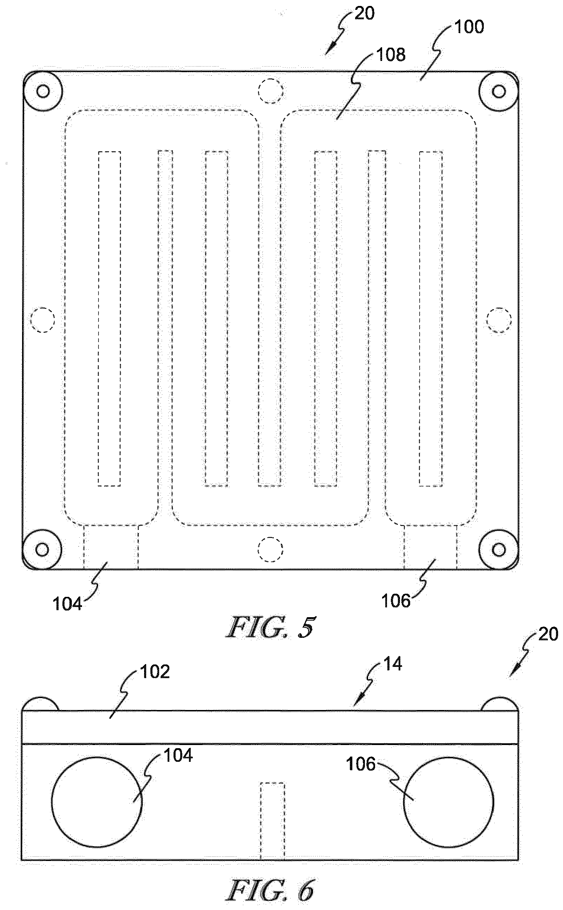

Referring now to Figs. 5-6, the thermoelectric coolers 20 are used as direct cooling or heating agents to create normothermia, hypothermia, or hyperthermia in a patient. The thermoelectric cooler 20 includes a body 100 having a top 102 that provides a thermal gradient. The skin contacting surface 14 extends across the top 102. The body 100 has a fluid inlet 104 and a fluid outlet 106. A fluid channel 108 extends through the body 100 between the fluid inlet 104 and the fluid outlet 106. Both the fluid inlet 104 and the fluid outlet 106 are fluidly coupled to the thermal exchanger 58. The liquid that is passed through the fluid channel 108 acts as a thermal reference for the thermoelectric coolers 20. Current is supplied by a controller to the thermoelectric coolers 20 to induce cooling or heating relative to the liquid. The liquid may be deionized water, e.g. deionized water with one or more additives to increase cooling performance, operational temperature zone, or corrosion properties, or another fluid with thermal conductivity properties. The thermoelectric coolers 20 operating as cooling devices remove heat from the user upon application of an applied electrical current. The thermoelectric coolers 20 may also be used as heating devices by reversal of the applied electrical current. Each thermoelectric cooler 20 includes a separate wired connection for an independent electrical current. Accordingly, some thermoelectric coolers 20 may be selectively used for cooling, and some thermoelectric coolers 20 may be selectively used for heating.

The controller 22 alters currents to each of the thermoelectric coolers 20 to control the temperature of each thermoelectric cooler 20 such that some of the thermoelectric coolers 20 are in cooling mode and others are in heating mode. A feedback loop on the controller 22 minimizes temperature variability. The cooling thermoelectric coolers 20 may be set to reach and maintain temperature at a point that reduces the risk of pressure injuries. In some embodiments, the optimal cooling temperature of the skin may be between approximately 30°C and 33°C. Cooling below 30°C may provide greater protection against pressure injury development; however, the loss of warming arising from greater cooling may offset the relatively smaller gains attained from cooling below 30°C. For example, reducing skin temperature from 33°C to 30°C may lead to a greater reduction in pressure injury risk than moving from 30°C to 27°C. All thermoelectric coolers 20 in the heat exchanger module 18 may be set to maintain a target temperature between approximately 15°C and 42°C. For example, the thermoelectric coolers 20 that are not in a cooling mode may be set to a warming mode between approximately 33°C to 42°C.

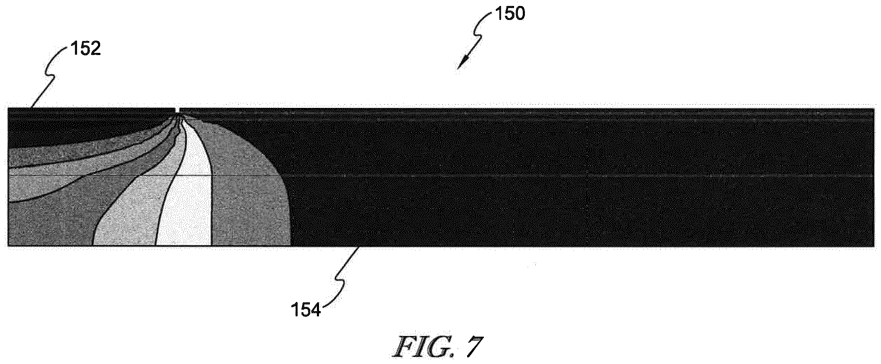

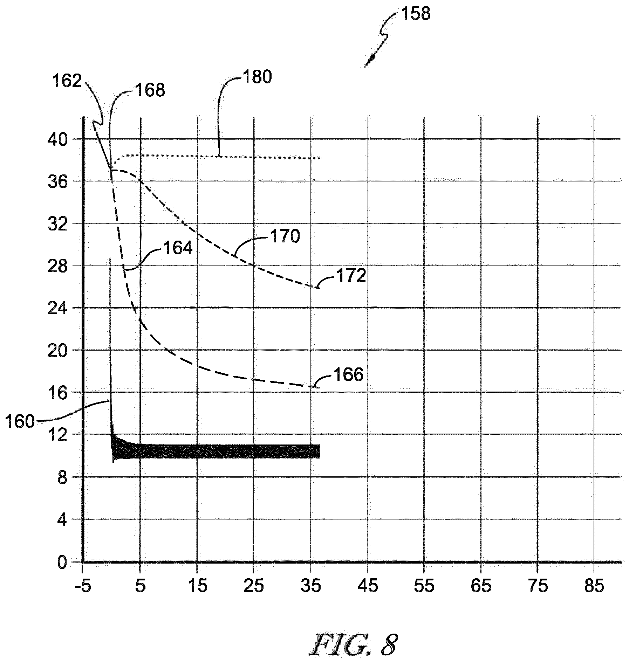

Referring to Figs. 7-8, a simulation model 150 of human tissue including skin, fat, muscle, and bone demonstrates that it is possible to maintain local cooling that is thermally distinct from heating in the periphery and beyond. It should be noted that the simulation is an extreme case, as typical cooling only needs to be a few degrees below average skin temperature of 32.7° C. The simulation model 150 simulates human tissue having a thickness of 100 millimeters. As illustrated in Fig. 7, cooling can be applied to a localized region 152 of the model 150, while heating is achieved in surrounding regions 154 of the model 150. Referring to Fig. 8, the graph 158 illustrates an amount of cooling required to remove heat from various thicknesses of body tissue. As illustrated by line 160, 37° C to 10° C cooling of the skin will cool approximately 1 millimeter of skin. 37° C cooling of fat will cool approximately 1 millimeter of fat as illustrated by point 162 of line 164. As shown in line 164, the amount of cooling required to remove heat from thicker fat increases with the thickness of the fat, e.g. as illustrated by point 166, 16° C of cooling is required to remove the heat from 35 millimeters of fat. 37° C cooling of bone will cool approximately 1 millimeter of bone as illustrated by point 168 of line 170. As shown in line 170, the amount of cooling required to remove heat from thicker bone increases with the thickness of the bone, e.g. as illustrated by point 172, 26° C of cooling is required to remove the heat from 35 millimeters of bone. 37° C warming of skin will heat approximately 1 millimeter to approximately 35 millimeters of skin as illustrated by line 180.

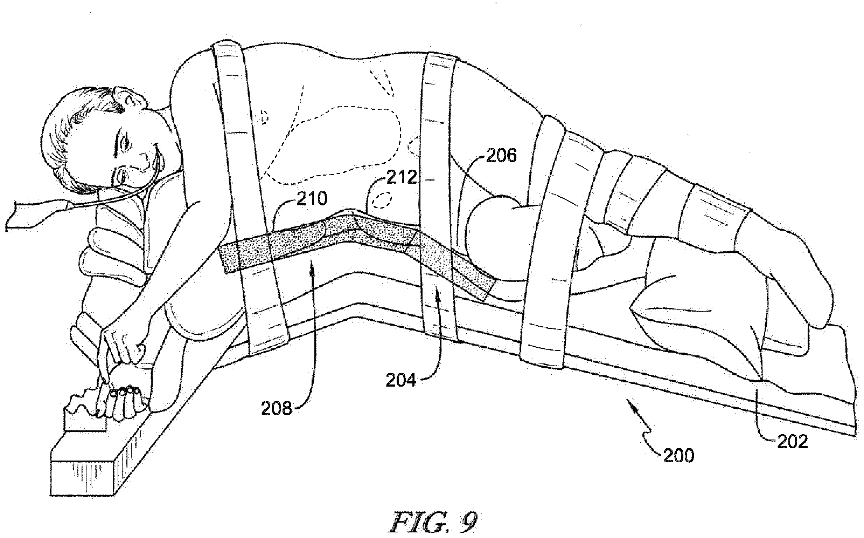

Fig. 9 is a perspective view of another embodiment of a surgical platform 200 having cushions 202. As illustrated in Fig. 9, the cooling zone 204 is positioned at the patient's hip 206. The heating zone 208 is positioned at the patient's shoulders 210 and side 212. Notably, as described herein, the cooling zones and heating zones may be positioned at any locations on the patient's body as desired.

The heat exchanger module 18 of the underbody warming device 16 provides the heat required to prevent perioperative hypothermia. Most perioperative warming devices on the market used to prevent perioperative hypothermia put out about 200 watts, and warming can be achieved with just 20% of the body surface area in contact with the warming device. Given an average body surface area of 1.8 m², 0.4 m² of body surface area delivers 200W at 0.5W/cm² of heating.

It should be noted that the embodiments described herein may be utilized for local cooling without warming to facilitate preventing pressure injuries. The embodiments may also be utilized for local warming for comfort with protective cooling. Moreover, the embodiments described herein may be used for local cooling for any purpose, such as comfort, relief from inflammation, burns, fever, perspiration, etc.

The embodiments described herein minimize the risk of pressure injuries originating in the operating, e.g. when bony prominences in contact with the surface of the surgical table are heated. Increased tissue temperatures increase the risk of pressure injuries. Accordingly, the embodiments described herein use thermoelectric coolers 20 for focal cooling of the bony prominences. Through use of the embodiments described herein, a solid working surface is regained by eliminating the puffy forced-air warming blanket underneath the surgical drape. Also, immediate warmth and comfort is provided to alert but apprehensive patients placed onto the operating table. Moreover, issues and concerns associated with the use of forced air blankets including the disruption of the sterile field are eliminated.

Following from the above description, it should be apparent to those of ordinary skill in the art that, while the methods and apparatuses herein described constitute exemplary embodiments, changes may be made to such embodiments.

Embodiments of the invention can be described with reference to the following numbered clauses, with preferred features laid out in the dependent clauses:
1. An underbody warming system comprising:
   a skin contacting surface configured to be positioned on the skin of a user, a plurality of thermoelectric devices,
   wherein a first thermoelectric device of the plurality of thermoelectric devices is positioned adjacent a first section of the skin contacting surface and has a first temperature different from the temperature of the skin being contacted by the first section of the skin contacting surface to create a first temperature gradient between the skin and the first thermoelectric device, wherein a second thermoelectric device of the plurality of thermoelectric devices is positioned adjacent a second section of the skin contacting surface and has a second temperature different from the first temperature and the temperature of the skin being contacted by the second section of the skin contacting surface to create a second temperature gradient between the skin and the thermoelectric device, and
   an insulator positioned between the first thermoelectric device and the second thermoelectric device,
   a first flow path configured to allow heat to flow between the skin and the first thermoelectric device, and
   a second flow path configured to allow heat to flow between the skin and the second thermoelectric device.
2. The underbody warming system of clause 1 further comprising at least one pressure sensor to detect a bony prominence of the user.
3. The underbody warming system of clause 2, wherein the second thermoelectric device is positioned at the bony prominence of the user.
4. The underbody warming system of and preceding clause, further comprising a controller to selectively operate the second thermoelectric device based on data from the pressure sensor.
5. The underbody warming system of any preceding clause further comprising a cushion layer positioned between the skin contacting surface and the plurality of thermoelectric devices.
6. The underbody warming system of any preceding clause, wherein the first temperature is greater than the second temperature.
7. The underbody warming system of any preceding clause, wherein the first temperature gradient provides heat to the skin being contacted by the first section of the skin contacting surface.
8. The underbody warming system of any preceding clause, wherein the second temperature gradient removes heat from the skin being contacted by the second section of the skin contacting surface.
9. The underbody warming system of any preceding clause, wherein the second section of the skin contacting surface is configured to be positioned at a bony prominence of the user.
10. The underbody warming system of any preceding clause, wherein at least one of the plurality of thermoelectric devices further comprises a thermometer to monitor at least one of a temperature of the thermoelectric device and a temperature of the skin.
11. The underbody warming system of clause 10, further comprising a controller to alter a current to the thermoelectric device in response to the temperature monitored by the thermometer.
12. The underbody warming system of any preceding clause, wherein the thermoelectric device includes a channel to circulate fluids through a cold sink of the thermoelectric device.
13. The underbody warming system of clause 12, further comprising a thermal exchanger to circulate fluids to and from the thermoelectric device.
14. The underbody warming system of any preceding clause, wherein the skin contacting surface is disposed within a surgical platform.
15. The underbody warming system of any preceding clause, further comprising a power supply positioned on the surgical platform.
16. An underbody warming system comprising:
   a skin contacting surface configured to be positioned on the skin of a user, a plurality of thermoelectric devices,
   wherein a first thermoelectric device of the plurality of thermoelectric devices is positioned adjacent a first section of the skin contacting surface and has a first temperature different from the temperature of the skin being contacted by the first section of the skin contacting surface to create a first temperature gradient between the skin and the first thermoelectric device, wherein the first temperature gradient provide heat to the skin being contacted by the first section of the skin contacting surface, and
   wherein a second thermoelectric device of the plurality of thermoelectric devices is positioned adjacent a second section of the skin contacting surface and has a second temperature different from the first temperature and the temperature of the skin being contacted by the second section of the skin contacting surface to create a second temperature gradient between the skin and the thermoelectric device, wherein the second temperature gradient removes heat from the skin being contacted by the second section of the skin contacting surface,
   a first flow path configured to allow heat to flow between the skin and the first thermoelectric device,
   a second flow path configured to allow heat to flow between the skin and the second thermoelectric device, and
   a cushion layer positioned between the skin contacting surface and the plurality of thermoelectric devices.
17. The underbody warming system of clause 16 further comprising at least one pressure sensor to detect a bony prominence of the user.
18. The underbody warming system of clause 17, wherein the second thermoelectric device is positioned at the bony prominence of the user.
19. The underbody warming system of any one of clauses 16 to 18, further comprising a controller to selectively operate the second thermoelectric device based on data from the pressure sensor.
20. The underbody warming system of any one of clauses 16 to 19, wherein the second section of the skin contacting surface is configured to be positioned at a bony prominence of the user.
21. The underbody warming system of any one of clauses 16 to 20, wherein at least one of the plurality of thermoelectric devices further comprises a thermometer to monitor at least one of a temperature of the flexible substrate and a temperature of the skin
22. The underbody warming system of clause 21, further comprising a controller to alter a current to the thermoelectric device based on the temperature monitored by the thermometer.
23. The underbody warming system of any one of clauses 16 to 22, wherein the thermoelectric device includes a channel to circulate fluids through a cold sink of the thermoelectric device.
24. The underbody warming system of clause 23, further comprising a thermal exchanger to circulate fluids to and from the thermoelectric device.
25. The underbody warming system of any one of clauses 16 to 24, wherein the skin contacting surface is disposed within a surgical platform.
26. The underbody warming system of clause 25, further comprising a power supply positioned on the surgical platform.
27. A method of providing localized cooling to the skin of an underbody warming system user, the method comprising:
   positioning a skin contacting surface on the skin of the user,
   positioning a first thermoelectric device adjacent a first section of the skin contacting surface,
   altering a temperature of the first thermoelectric device to create a first temperature gradient between the skin and the first thermoelectric device,
   positioning a second thermoelectric device adjacent a second section of the skin contacting surface,
   altering a temperature of the second thermoelectric device to create a second temperature gradient between the skin and the second thermoelectric device,
   providing a first flow path through the first thermoelectric device to allow heat to flow between the skin and the first thermoelectric device, and
   providing a second flow path through the second thermoelectric device to allow heat to flow between the skin and the second thermoelectric device.
28. The method of clause 27 further comprising detecting a bony prominence of the user with a pressure sensor.
29. The method of clause 28, further comprising positioning the second thermoelectric device at the bony prominence of the user.
30. The method of clause 29, further comprising selectively operating the second thermoelectric device based on data from the pressure sensor.
31. The method of clause 27, further comprising positioning a cushion layer between the skin contacting surface and the first thermoelectric device.
32. The method of clause 31, further comprising positioning the cushion layer between the skin contacting surface and the second thermoelectric device.
33. The method of clause 27, further comprising altering the temperature of the first thermoelectric device to a temperature that is greater than the temperature of the second thermoelectric device.
34. The method of clause 27, further comprising providing heat to the skin being contacted by the first section of the skin contacting surface through the first temperature gradient.
35. The method of clause 34, further comprising removing heat from the skin being contacted by the second section of the skin contacting surface through the second temperature gradient.
36. The method of clause 35, further comprising positioning the second section of the skin contacting surface at a bony prominence of the user.
28. The method of clause 21, further comprising monitoring a temperature of the skin.
29. The method of clause 28, further comprising altering a current to the thermoelectric device in response to the temperature monitored by the thermometer.
30. The method of clause 21, further comprising circulating fluids from a thermal exchanger through a cold sink of the first thermoelectric device.
31. The method of clause 21, further comprising circulating fluids from a thermal exchanger through a cold sink of the second thermoelectric device.
32. The method of clause 21, further comprising disposing the skin contacting surface within a surgical platform.
33. The method of clause 32, further comprising disposing a power supply on the surgical platform.

## Claims

1. An underbody warming system comprising:
a skin contacting surface configured to be positioned on the skin of a user,
a plurality of thermoelectric devices,
wherein a first thermoelectric device of the plurality of thermoelectric devices is positioned adjacent a first section of the skin contacting surface and has a first temperature different from the temperature of the skin being contacted by the first section of the skin contacting surface to create a first temperature gradient between the skin and the first thermoelectric device,
wherein a second thermoelectric device of the plurality of thermoelectric devices is positioned adjacent a second section of the skin contacting surface and has a second temperature different from the first temperature and the temperature of the skin being contacted by the second section of the skin contacting surface to create a second temperature gradient between the skin and the thermoelectric device, and
an insulator positioned between the first thermoelectric device and the second thermoelectric device,
a first flow path configured to allow heat to flow between the skin and the first thermoelectric device, and
a second flow path configured to allow heat to flow between the skin and the second thermoelectric device.

2. The underbody warming system of claim 1 further comprising at least one pressure sensor to detect a bony prominence of the user.

3. The underbody warming system of claim 2, wherein the second thermoelectric device is positioned at the bony prominence of the user.

4. The underbody warming system of any preceding claim, further comprising a controller to selectively operate the second thermoelectric device based on data from the pressure sensor.

5. The underbody warming system of any preceding claim further comprising a cushion layer positioned between the skin contacting surface and the plurality of thermoelectric devices.

6. The underbody warming system of any preceding claim, wherein the first temperature is greater than the second temperature.

7. The underbody warming system of any preceding claim, wherein the first temperature gradient provides heat to the skin being contacted by the first section of the skin contacting surface.

8. The underbody warming system of any preceding claim, wherein the second temperature gradient removes heat from the skin being contacted by the second section of the skin contacting surface.

9. The underbody warming system of any preceding claim, wherein the second section of the skin contacting surface is configured to be positioned at a bony prominence of the user.

10. The underbody warming system of any preceding claim, wherein at least one of the plurality of thermoelectric devices further comprises a thermometer to monitor at least one of a temperature of the thermoelectric device and a temperature of the skin.

11. The underbody warming system of claim 10, further comprising a controller to alter a current to the thermoelectric device in response to the temperature monitored by the thermometer.

12. The underbody warming system of any preceding claim, wherein the thermoelectric device includes a channel to circulate fluids through a cold sink of the thermoelectric device.

13. The underbody warming system of claim 12, further comprising a thermal exchanger to circulate fluids to and from the thermoelectric device.

14. The underbody warming system of any preceding claim, wherein the skin contacting surface is disposed within a surgical platform.

15. The underbody warming system of claim 14, further comprising a power supply positioned on the surgical platform.
